# EUROPEAN PATENT SPECIFICATION

(11) **EP 2 756 005 B1**
(45) Date of publication and mention of the grant of the patent: **02.03.2016**
(21) Application number: 12772367.4
(22) Date of filing: 11.09.2012
(51) Int. Cl.: C07K 17/14, G01N 33/543, G01N 33/574, G01N 33/68, G01N 33/569, C12N 5/00, B03C 1/00

(54) **INTRACELLULAR CELL SELECTION**
INTRAZELLULÄRE ZELLENAUSWAHL
SÉLECTION DE CELLULES INTRACELLULAIRES

(30) Priority: 14.09.2011 GB 201115847; 24.04.2012 GB 201207177
(43) Date of publication of application: 23.07.2014
(73) Proprietor: Abeterno Technologies Limited, Cambridge CB22 3GN (GB)
(72) Inventor: CODRINGTON, Rosalind, Impington CB24 9NF (GB)
(74) Representative: Docherty, Robert Charles
(86) International application number: PCT/GB2012/052227
(87) International publication number: WO 2013/038158

(56) References cited:
- WO-A1-97/08557
- WO-A2-2005/081721
- SMITH C A M ET AL: "The effect of static magnetic fields and tat peptides on cellular and nuclear uptake of magnetic nanoparticles", BIOMATERIALS, ELSEVIER SCIENCE PUBLISHERS BV., BARKING, GB, vol. 31, no. 15, 1 May 2010 (2010-05-01), pages 4392-4400, XP026971517, ISSN: 0142-9612 [retrieved on 2010-02-26] cited in the application

## Description

### Introduction

This disclosure relates to the selection and isolation of mammalian cells from a heterogeneous population of mammalian cells by affinity binding of intracellular antigens by ligands, typically antibody fragments, and the use of magnetic particles to select said cells.

### Background

Selective cell separation is an essential method in experimental biology and medicine. It is an increasingly important process that is driven by the ever increasing demands for sensitivity, selectivity, yield, time and economy of the process. The most popular aim for cell separation is to achieve complete removal of the target population from a cell mixture. However, for some applications, a secondary aim may be to recover the selected cells for study for additional manipulation. Additionally, the purity of separation may be more important than the yield of the selected cells, or vice versa. Thus, the aims for cell separation determine the method that is chosen to achieve the separation. Other factors to take into account when selecting the separation technique are the processing time, viability of residual cells, and exposure to non-medically approved reagents.

There are a number of cell separation techniques which can be broadly classified into two categories: techniques based on size and density, and techniques based on affinity (chemical, electrical, or magnetic) (Radisic *et al,* 2006).

Magnetic separation techniques are a common method in many laboratories and have been broadly used for targeted drug delivery, imaging and bioseparation such as in selective cell separations, antibody purification, protein affinity purification, immunoprecipitations, protein fractionations, organelle isolations, total nucleic acid purification and polyadenylated mRNA purification (Saiyed *et al.,* 2003; Nord *et al.,* 2001; Stark *et al.,* 1988; Suzuki *et al.,* 1996; Weissleder *et al.,* 1997). For example, WO97/08557 discloses the separation of carcinoma cells from the peripheral blood by magnetic sorting. Therefore tumor cells are magnetically labelled with antibodies directed to tissue specific antigens, preferably cytoplasmatic proteins. In order for the magnetic particle to reach the intracellular antigen, the cells should be permeable. The cells are subsequently separated on a magnetic matrix.

Smith et al [Biomaterials Vol 31(15) p4392-4400] reviews two methods facilitating cell entry of magnetic particles using external magnetic fields or functionalising the nanoparticles with cell penetrating peptides (e.g. tat), suggesting functionalised NP to be the preferred method for cellular and nuclear delivery opposed to using typical magnetofection techniques.

WO2005/081721 discloses semiconductor nanoparticle complexes in association with cationic polymers and methods for enhancing the transport of semiconductor nanoparticles across biological membranes using cationic peptides.

The magnetic cell separation technique consists of magnetic beads coated with antibodies specific for cell surface antigens on the desired cells. When the antibody-magnetic beads are exposed to a mixed cell population, they attach to the surface of the desired cells via antibody-antigen interaction. The desired cell subpopulation can then be separated in the presence of a strong magnetic field. The antibodies used in these applications are typically monoclonal antibodies specific for cell surface antigens. However, antibody fragments such as single chain antibodies (scFv) have also been successfully coupled to magnetic beads and used in a variety of applications such as phage display libraries of scFv and in imaging of tumours (McConnell *et al.,* 1999; Nord *et al.,* 2001; Han *et al.,* 2006).

Single VH and VL domains are the smallest functional modules of antibodies required for antigen binding. ScFv are a fusion of the variable regions of the heavy (VH) and light chains (VL) of immunoglobulins, connected with a short linker peptide of 10 to 25 amino acids. Their small size conveys them with distinct advantages over whole antibodies, in particular they have rapid pharmacokinetics, greater tumour penetration and lower immunogenicity than intact IgG, F(ab')₂, or Fab (Yokota *et al.,* 1992). They have also shown to be particularly useful in clinical applications involving the selective delivery of radionucleotides to tumours (Huston *et al.,* 1996; Reisfeld *et al.,* 1996; Laske *et al.,* 1997). The scFv may be assembled from the variable regions of particular monoclonal antibodies (Huston *et al.,* 1988; Brinkmann *et al.,* 1991) or made *de novo* from phage display libraries (Winter *et al.,* 1994; Chester *et al.,* 1994). They retain the specificity of the original immunoglobulin, and in many applications outperform whole IgG in construction speed, production yield, and engineering flexibility, as they can be easily expressed in bacterial expression systems. Indeed, many scFv have been produced in bacteria with good yields, either as insoluble inclusion bodies in *Escherichia coli* (Huston et al., 1988) or by secretion into the periplasm (Skerra et al., 1993) or into the culture supernatant (Takkinen et al., 1991).

A major caveat of magnetic cell separation is that it is only applicable to cells that can be separated by their specific surface antigens. This can be particularly limiting when the cell specific markers are mainly found intracellularly and are not readily available on the cell surface. Indeed, in most cancers, the cancer markers of interest are predominantly expressed inside the cells and therefore cannot be used for cell separations. The inherent difficulties surrounding cell internalisation of antibodies have made it difficult so far to use intracellularly expressed antigens in cell separations. Whole monoclonal antibodies cannot be internalised or be expressed in cells due to the reducing environment of the cell.

However, scFv are routinely stably expressed in cells and have been reportedly been able to internalise into cells with the help of carrier mediated systems such as transduction peptides (Futaki *et al.,* 2001; Avignolo *et al.,* 2008; Niesner *et al.,* 2002; Ma *et al.,* 2011). In addition, recent studies using magnetic particles have shown that it is possible to internalise these particles into cells and control their location by means of an external magnet (Tseng *et al.,* 2009). Moreover, they have been used in cancer therapeutics as a means to direct the cells containing anti-cancer agents to a desired location on the cancer cells by means of an external magnetic field, thus, promising to reduce the cytotoxic effects of drugs by increasing the bioavailability of therapeutic compounds at the site of action and improving pharmacokinetics (Cinti *et al.,* 2011). Magnetic beads have also been used as a means to internalise nucleic acids (magnetofection) (Scherer *et al.,* 2002) and small peptides into cells (Han *et al.,* 2006) and have been previously coupled to cationic peptides (Smith *et al.,* 2002) to enhance their uptake *in vivo* and allow their use for MRI scanning (Stark *et al.,* 1988; Suzuki *et al.,* 1996; Weissleder *et al.,* 1997). Additionally they have been coupled to drugs for magnetic drug targeting in patients with solid tumours (Lubbe *et al.,* 1996; Alexiou *et al.,* 2000). Furthermore, these magnetic particles do not rely on receptors or other cell membrane bound proteins for their cellular uptake, it is possible to transfect cells that are normally non-permissive (Scherer *et al.,* 2002). This is also true in primary cells which are known to be difficult to transfect (Krotz *et al.,* 2003).

### Statements of Invention

The present disclosure is directed to overcoming the deficiencies in magnetic cell separation by providing a method to separate a target population from a cell mixture on the basis of the expression of their intracellular markers and to then allow the recovery of these cells for additional downstream studies and to achieve therapeutic benefits.

This disclosure relates to a method for isolating specific cells from a mixed population of different cell types using intracellular markers rather than cell surface markers. This method involves the coupling of antibody fragments to a magnetic particle, where the antibody fragment is specific to the marker of interest expressed inside the cell.

According to an aspect of the invention there is provided a magnetic particle comprising: a ligand that specifically binds an intracellular antigen, a cell membrane penetrating cationic peptide and an export peptide comprising an amino acid sequence that is adapted to interact with the secretory pathway in a cell.

In a preferred embodiment of the invention said intracellular antigen is substantially a nuclear antigen.

In a preferred embodiment of the invention said nuclear antigen is selected from the group consisting of: FoxO family members, TARP, p53, Rb, E2F, hK4, BRCA1, Cdk2, SATB1, TP53INP1, Myc, Fos, Jun, CREB, Ets, SRF, FAK, Pax6, Calpain, Elk1, Stats 1-3, Akt, p21

In an alternative preferred embodiment of the invention said intracellular antigen is substantially a cytoplasmic antigen.

In a preferred embodiment of the invention said cytoplasmic antigen is selected from the group consisting of: Jak1, Jak2, Tyk2, Jak3, GATA 1-4, Stats 1-6, CBP, NFkB, IKK, PIK3CA, B-raf, EBI3, eIF2α, Akt, PI3K, IAP, Hsp, FAK, Raf, Ras, TNF, Src, Abl, Caspases 1-12.

In a preferred embodiment of the invention said cationic cell membrane penetrating peptide is a natural cell penetrating peptide.

In an alternative preferred embodiment of the invention said cell membrane penetrating peptide is a synthetic sequence.

In a preferred embodiment of the invention said cell membrane penetrating peptide is adapted to penetrate a mammalian cell.

In a preferred embodiment of the invention said cell membrane penetrating peptide is selected from the group consisting of: YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ, RRRRRRRRRRRR.

In a preferred embodiment of the invention said cell membrane penetrating peptide consists essentially of the amino acid sequence: GRKKRRQRRRPQ.

Preferably said cell membrane penetrating peptide consists of the amino acid sequence: GRKKRRQRRRPQ.

In a preferred embodiment of the invention said cell membrane penetrating peptide consists essentially of the amino acid sequence: RRRRRRRRRRRR.

Preferably said cell membrane penetrating peptide consists of the amino acid sequence: RRRRRRRRRRRR.

The cell penetrating peptide and the secretory peptide can be engrafted into the CDR3 regions of the VL and VH domains.

In a preferred embodiment of the invention said peptide adapted to interact with the mammalian secretory pathway is selected from the group consisting of: MCPARSLLLVATLVLLDHLSLA,MDAMKRGLCCVLLLCGAVFVSPS,MATGSRTSLL LAFGLLCLPWLQEGSA, MYRMQLLSCIALSLALVTNS or MGVKVLFALICIAVAE.

In a preferred embodiment of the invention said peptide is consists essentially of the amino acid sequence MGVKVLFALICIAVAE.

In a preferred embodiment of the invention said peptide is consists of the amino acid sequence: MGVKVLFALICIAVAE.

In a preferred embodiment of the invention said peptide is consists essentially of the amino acid sequence: MYRMQLLSCIALSLALVTNS.

In a preferred embodiment of the invention said peptide is consists of the amino acid sequence: MYRMQLLSCIALSLALVTNS.

In a preferred embodiment of the invention said ligand that binds said intracellular antigen is an antibody fragment.

Various fragments of antibodies are known in the art, e.g. Fab, Fab₂, F(ab')₂, Fv, Fc, Fd, scFvs, etc. A Fab fragment is a multimeric protein consisting of the immunologically active portions of an immunoglobulin heavy chain variable region and an immunoglobulin light chain variable region, covalently coupled together and capable of specifically binding to an antigen. Fab fragments are generated via proteolytic cleavage (with, for example, papain) of an intact immunoglobulin molecule. A Fab₂ fragment comprises two joined Fab fragments. When these two fragments are joined by the immunoglobulin hinge region, a F(ab')₂ fragment results. An Fv fragment is multimeric protein consisting of the immunologically active portions of an immunoglobulin heavy chain variable region and an immunoglobulin light chain variable region covalently coupled together and capable of specifically binding to an antigen. A fragment could also be a single chain polypeptide containing only one light chain variable region, or a fragment thereof that contains the three CDRs of the light chain variable region, without an associated heavy chain variable region, or a fragment thereof containing the three CDRs of the heavy chain variable region, without an associated light chain moiety; and multi specific antibodies formed from antibody fragments, this has for example been described in US patent No 6,248,516. Fv fragments or single region (domain) fragments are typically generated by expression in host cell lines of the relevant identified regions. These and other immunoglobulin or antibody fragments are within the scope of the invention and are described in standard immunology textbooks such as William E. Paul, Fundamental Immunology or Janeway et al. Immunobiology (cited above). Molecular biology now allows direct synthesis (via expression in cells or chemically) of these fragments, as well as synthesis of combinations thereof. A fragment of an antibody or immunoglobulin can also have bispecific function as described above.

Methods to deliver antibody fragments to cells intracellularly are known in the art; for example see WO2007/064727; WO2004/030610; WO03/095641; WO02/07671; WO01/43778; WO96/40248; and WO94/01131 each of which is incorporated by reference in their entirety.

In a preferred embodiment of the invention said antibody fragment is an ScFv or a single VH or VL domain.

In an alternative preferred embodiment of the invention said ligand that binds said intracellular antigen is a peptide or a peptide aptamer.

Peptides that have binding affinity to a target antigen are within the scope of the invention. For example peptides that mimic the binding affinity of antibodies and antibody fragments; see Chattophadayay, A, Tate, S., Beswick, R., Wagner, S.D. and Ko Ferrigno, P. A peptide aptamer to antagonise BCL-6 function. Oncogene, 25 2223-2233 (2006).

In a preferred embodiment of the invention said cell is a mammalian cell.

In a preferred embodiment of the invention said magnetic particle further comprises a nuclear export peptide.

In a preferred embodiment of the invention said nuclear export peptide comprises or consists essentially of the amino acid sequence: NELALKLAGLDINKTEGEEDAQ.

According to a further aspect of the invention there is provided a method for the selection and isolation of a mammalian cell from a heterogeneous population of cells comprising:
i) forming a first preparation comprising a heterogeneous population of mammalian cells;
ii) contacting said population with a second preparation comprising a magnetic particle according to the invention to provide a combined preparation and providing conditions that allow penetration and binding of said magnetic particle to an intracellular antigen;
iii) incubating said combined preparation to remove unbound magnetic particles by the secretory pathway of said mammalian cell, optionally by application of a magnetic field; and
iv) applying a magnetic field to said combined preparation to select and isolate mammalian cells comprising magnetic particles bound to an intracellular antigen.

In a preferred method of the invention said mammalian cell is a stem cell.

Preferably said stem cell is a cancer stem cell.

The concept of a cancer stem cell within a more differentiated tumour mass, as an aberrant form of normal differentiation, is now gaining acceptance over the current stochastic model of oncogenesis, in which all tumour cells are equivalent both in growth and tumour-initiating capacity (Hamburger *et al.,* 1977; Pardal *et al.,* 2003). For example, in leukaemia, the ability to initiate new tumour growth resides in a rare phenotypically distinct subset of tumour cells (Bonnet *et al.,* 1997) which are defined by the expression of CD34+CD38 surface antigens and have been termed leukemic stem cells (LSC). Similar tumour-initiating cells have also been found in 'solid' cancers such as prostate (Collins *et al.,* 2005), breast (AlHajj *et al.,* 2003), brain (Singh *et al.,* 2004), lung (Kim *et al.,* 2005), colon (O'Brien *et al.,* 2007; Ricci Vitiani *et al.,* 2007) and gastric cancers (Houghton *et al.,* 2004). A list of intracellular cancer stem cell markers is available in Table 1.

According to an aspect of the invention there is provided a kit for customized modification of magnetic particles containing a selection of cell membrane penetrating cationic peptides selected from the group of YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ, RRRRRRRRRRRR, a selection of secretory proteins selected from the group MCPARSLLLVATLVLLDHLSLA, MDAMKRGLCCVLLLCGAVFVSPS, MATGSRTSLLLAFGLLCLPWLQEGSA, MYRMQLLSCIALSLALVTNS or MGVKVLFALICIAVAE and activation, wash and blocking/storage buffer.

In a preferred embodiment of the invention said kit further comprises a magnet for the use of said magnetic particles.

The magnetic particles may be coupled to an antibody fragment (scFv, single domain, dibody), a cell penetrating peptide and a secretory peptide, or to an antibody fragment and a secretory peptide.

The cell penetrating peptide and the secretory peptide may be either directly coupled to the magnetic particle or to the antibody fragment via expression as a fusion protein or via chemical coupling between the antibody and the peptide, or they may be engrafted within the CDR3 regions of the VH or VL domains as previously described by (Jeong *et al.* 2011).

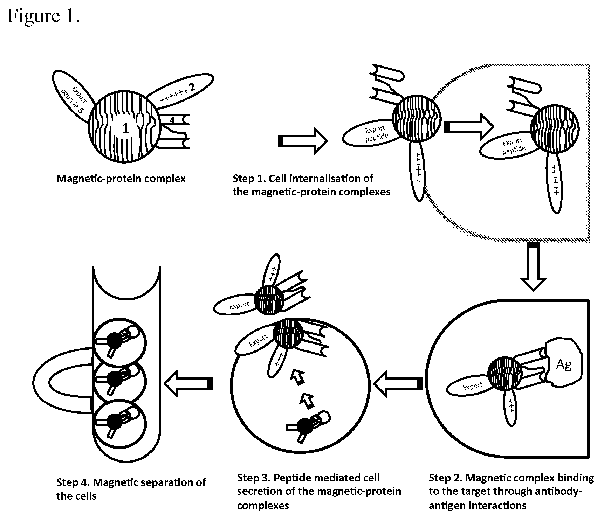

The magnetic antibody fragments are incubated with the cells and allowed to internalize into the cells using the cationic peptide or the positive charge of the magnetic particle as the delivery vehicle. After achieving cell internalisation of the antibody magnetic particles, the cells are allowed a period of incubation in cell media to allow the magnetic antibody fragments bind to their targets. Any unbound magnetic antibody fragments will then be secreted from the cells by the secretory peptide with or without the help of an external magnet. The cells containing just the bound antibody fragments to their respective targets can be selected with the use of an external magnet where any unbound cells are washed away. The selected cells can then be further incubated for other downstream applications (Figure 1). Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

**Table 1. Intracellular Cancer stem cell markers**

| | | |
|---|---|---|
| TP53INP1 | tumor protein p53 inducible nuclear protein 1 | ENSG00000164938 |
| Axl | AXL receptor tyrosine kinase | ENSG00000167601 |
| GATA-3 | GATA binding protein 3 | ENSG00000107485 |
| USP22 | ubiquitin specific peptidase 22 | ENSG00000124422 |
| BIRC6 | baculoviral IAP repeat containing 6 | ENSG00000115760 |
| Hh | sonic hedgehog | ENSG00000164690 |

Figure 1: Diagram depicting the sequence of events in the magnetic intracellular cell separation technique (MICELS). A magnetic particle (1) is coupled to a cell penetrating positively charged peptide (2), a cell export peptide (3) and an scFv antibody fragment (4). Step 1. The magnetic-protein complex is incubated with the mixed cell population for a length of time until the complex has fully internalised into the cells. Step 2. The scFv antibody fragments specifically bind to their antigen targets (Ag) available in the cells and are thereby anchored inside these cells. Step 3. Any magnetic-protein complexes that are not bound to the specific target antigens are secreted to the extracellular milieu via the export peptide through the cell secretory pathway. This step avoids the selection of cells that do not express the specific target antigens. Step 4. An external magnetic field is applied to the cells allowing only the cells containing the magnetic complexes to bind to the magnet. The remainder of the cell population can be washed away. The selected cells can be now used for further downstream applications.

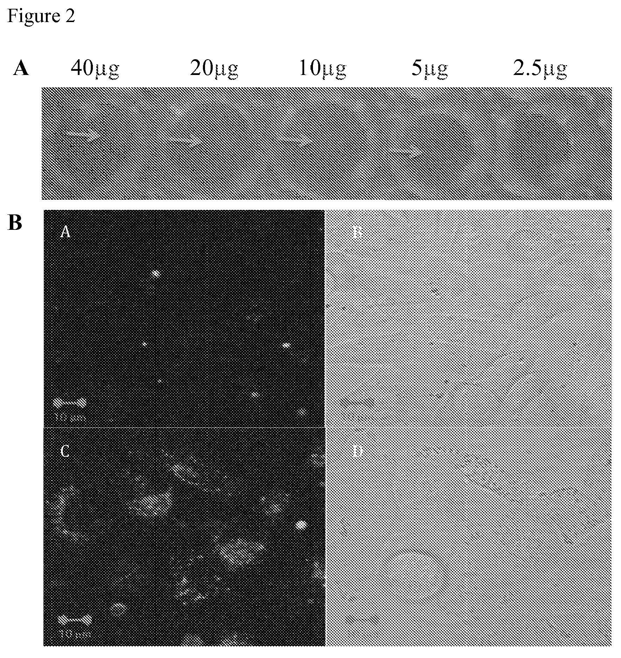

Figure 2 MICELS selection of CHO and A549 cells with scFv magnetic-protein complexes. (A) MICELS selection was performed on A549 cells in a 10cm dish with an excess of the scFv magnetic-protein complexes (200µg). The cells were washed with PBS, trypsinised and selected using the magnet. The selected cells containing the excess scFv magnetic-protein complexes (white-red pellet) can be seen on top of each magnet. (B) Confocal microscopy on CHO cells (A, B) and A549 cells (C, D) incubated with an excess of scFv magnetic-protein complexes. The cells were vigorously washed with PBS before imaging. The green complexes can be seen localised in the cytoplasm of the cells.

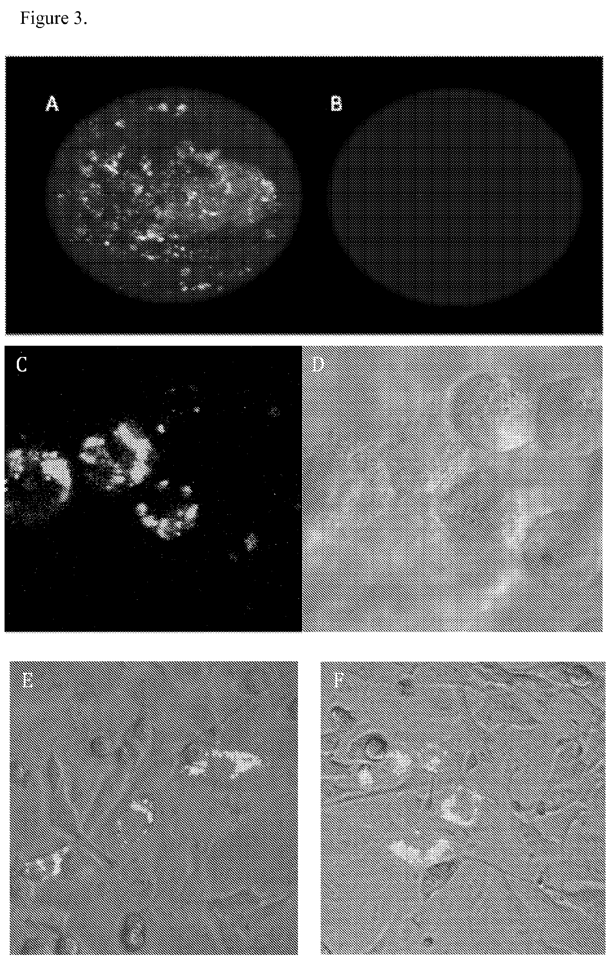

Figure 3 MICELS selection of CHO and A549 cells with scFv magnetic-protein complexes. (A) MICELS selection of A549 cells in a 24 well plate with a serial dilution of the scFv magnetic-protein complexes (40µg-2.5µg). The blue arrows indicate the location of the selected cell pellets. Decreasing amounts of selected cells were obtained with decreasing concentrations of magnetic-protein complexes. The best results for cell selection were obtained with 40µg of the magnetic-protein complexes. (B) Confocal images of CHO (A, B) and A549 (C, D) cells after 4.30h incubation with scFv magnetic-protein complexes. The cells were washed vigorously with PBS before imaging. The green magnetic-protein complexes localised at the cell membrane, the perinuclear membrane and in the cytoplasm consistent with the sub-cellular localisation of activated Ras in the A549 lung cancer cell line.

Figure 4 MICELS selection of A549 and CHO cells with 40µg scFv magnetic-protein. Images of A549 cells (A) and CHO cells (B) selected using MICELS were imaged using a 10x objective. The images were taken after an extensive wash with PBS followed by trypsinisation and cell selection with the magnet. Minimal background of CHO cells was observed following MICELS selection. (C) Confocal image of A549 cells at 24h in culture following MICELS selection.

### Materials and Methods:

### Production of H-RasG12V scFv

The sequence of an scFv (scFv#6) specific for the mutant H-RasG12V (Tanaka et al., EMBO Journal 2007) served as a template for the synthesis of scFv#6 by GeneArt® (Life Technologies) with flanking BamH1 and EcoR1 sites at the 5' and 3' ends, respectively. The gene was subcloned into BamH1 and EcoR1 sites of the pRSETa bacterial expression vector (Life Technologies) to generate in-frame fusion scFv protein with a 6x histidine tag. The plasmid was transformed into BL21(DE3) bacterial cells (Merck-Millipore) and expression of the scFv#6 fragment was induced in 500ml culture via the addition of 1 mM IPTG at OD₆₀₀ of 0.55 followed by incubation for 3 h at 37°C. The cells were harvested by centrifugation at 5000 × g at 4°C and the final cell pellets were stored at -80°C prior to scFv purification.

scFv protein was extracted from bacterial pellets in 25ml of B-PER bacterial lysis buffer (Thermo Scientific) in the presence of a cocktail of protease inhibitors (containing 1.5 µg/ml Chymotrypsin, 0.8 µg/ml Thermolysin, 1 mg/ml Papain, 1.5 µg/ml Pronase, 1.5 µg/ml Pancreatic extract, 0.002 µg/ml Trypsin) (Roche) at RT for 30min. The lysate was centrifuged at 15000 × g at 4°C and the supernatant was diluted in 25ml lysis buffer (50 mM Na phosphate, pH 8.5, 300 mM NaCl, 10 mM imidazole). The His-tagged scFv#6 proteins were purified by gravity flow through 1ml of Ni-NTA agarose (Qiagen), followed by a wash with 20ml of wash buffer (50 mM Na phosphate, pH 8.5, 300 mM NaCl, 20 mM imidazole) and eluted in 5ml of elution buffer (50 mM Na phosphate, pH 8.5, 300 mM NaCl, 250 mM imidazole). The eluted protein was subsequently dialysed against 0.1M MES pH6.0, snap frozen and stored at -80°C at a concentration of 1.4mg/ml

### Magnetic Particles

Biodegradable magnetic nanoparticles with an average size of 100 nm (by dynamic light scattering) were purchased from Chemicell, Berlin, Germany. These particles are referred to as nano-screenMAG particles with an extension to their name -ARA, referring to the surface coating of the iron oxide core with Glucuronic acid used for the binding to biomolecules, and are covered by a lipophilic fluorescence dye emitting light in the range of green (Exc.= 476 nm / Emₘₐₓ.= 490 nm).

### Coupling of fluorescent magnetic particles to proteins

FITC labelled Tat cell penetrating peptide 5(6)-Carboxyfluorescein-GRKKRRQRRRPQ and Gaussia Luciferase secretory peptide 5(6)-Carboxyfluorescein-MGVKVLFALICIAVAEA were synthesised by JPT Peptide Technologies GmbH. The lyophilised peptides were resuspended in 100µl of DMSO and subsequently diluted down to a concentration of 1mg/ml with 10mM PBS pH7.4, snap frozen and stored at -80°C in aliquots.

The magnetic particles were activated by 2 washes with 1ml 0.1M MES buffer pH5.0 using the MagnetoPure (Chemicell, Berlin, Germany) magnetic separator. After the second wash the particles were resuspended in 0.25ml 0.1M MES buffer pH5.0. The carboxyl groups on the green fluorescent magnetic beads nano-screenMAG/G-ARA were activated with freshly prepared EDC (1-Ethyl-3-(3-dimethylaminopropyl)carbodiimide) (Sigma) by dissolving 10mg EDC in 0.25ml 0.1M MES buffer. The EDC buffer was added to the particles and gently mixed at RT for 10min. Following this incubation, the magnetic particles were washed 2 x with 1ml 0.1M MES buffer pH5.0 and resuspended in 0.25ml of 0.1M MES buffer, pH5.0.

200µg of scFv#6 purified protein were mixed with 150µg of Tat cell penetrating peptide and 200µg of Gaussia luciferase export peptide in 0.25ml of 0.1M MES buffer, pH 5.0. The protein mixture was added to 10mg of activated particles and gently mixed for two hours at room temperature to generate magnetic-protein complexes. The particles were washed 3 x with 1 ml PBS and resuspended in 500µl Blocking/Storage buffer (10mM PBS pH7.4, 0.1 % BSA, 0.05 % sodium azide).

### Cell Culture

Chinese Hamster Ovary (CHO) cells and the A549 human lung carcinoma cell line (which constitutively express mutant K-RasG12V antigen) were maintained in selective Gibco® Ham's F12 Nutrient Mixture Media (Life Technologies) supplemented with 10% heat-inactivated foetal bovine serum, 100 µg/mL streptomycin, 0.29 mg of L-glutamine at 37°C in a humidified atmosphere of 5% CO₂.

### Magnetic intracellular cell separation technique (MICELS)

The CHO and A549 cell lines were seeded the day before the magnetic selection, at 2x10⁵ cells/well in a 24 well glass bottom plate (In Vitro Scientific) in 1ml of growth media to allow the cells reach 90-95% confluency at the time of the magnetic cell separation. The following day the media was replaced with fresh growth media pre-warmed at 37°C. A serial dilution ranging between 40µg and 2.4µg of the magnetic-protein mix was gently mixed with 10µl of serum free Ham's F12 media and added drop wise to the cells. The cells were incubated with the magnetic-protein complexes for 1.30h at 37°C in a humidified atmosphere of 5% CO₂ to allow these to transduce into the cells. The cells were then vigorously washed five times with PBS and incubated further for 3h. Following the 3 hour incubation, any unbound magnetic-protein complexes are secreted from the cells through the cell secretory pathway. The cells were then vigorously washed five times with PBS and trypsinised in the 24 well plate with 0.05% trypsin-EDTA (Invitrogen). 0.5ml of fresh F12 growth media was added to each well to inactivate the Trypsin and the 24 well plate was placed on top of a MagnetoPURE (Chemicell, Berlin, Germany) magnetic separator for cell selection. At this point any cells that did not contain magnetic-protein complexes were gently washed away 3 times with 500µl PBS on the magnetic separator. The magnetic separator was then removed and the remaining cells containing the magnetic-scFv complexes bound to the activated form of Ras were further incubated in 500µl of growth media at 37°C in a humidified atmosphere of 5% CO₂ for 48h.

### Image acquisition Laser scanning Confocal microscopy.

Laser scanning confocal microscopy was carried out using an Inverted Zeiss LSM 510 META Axiovert 200M confocal microscope using a 10x and a 40x/1.3NA oil immersion objective. FITC was imaged using an Argon 488-nm laser light and a 505-530-nm BP emission filter

### EXAMPLES

A method to separate whole live cells using intracellular proteins rather than cell surface proteins (MICELS-magnetic intracellular cell separation) was developed by coupling magnetic particles to scFv antibody fragments and to cell penetrating and cell export peptides (Figure 1). In this study the proto-oncogene mutant RasG12V was used as a model system to test the MICELS technology. The Ras protein belongs to a class of proteins known as small GTPases, and plays key roles in signal transduction as molecular switches. These proteins are mediated through two switch regions displaying conformational differences between active (GTP bound) and inactive (GDP bound) states (Vetter and Wittinghofer, 2001). Ocassionally, mutations occur in these Ras proteins (K, H or NRAS) resulting in constitutively activated GTP-bound forms that promote cell transformation in a signal-independent manner (Adjei, 2001), thereby, promoting cell proliferation and protection from apoptosis. Approximately 30% of human cancers contain oncogenic Ras mutants, with higher frequencies in pancreas, colon and lung adenocarcinoma (Grewal *et al.,* 2011). The Ras isoforms (H-Ras, N-Ras and K-Ras) are mainly localised to the inner cell membrane, however, in recent years they have been shown to associate, as well, with intracellular organelles such as the Golgi, the ER, endosomes and the mitochondria (Grewal *et al.,* 2011).

An scFv antibody fragment (scFv#6) previously shown to bind specifically to the activated form of Ras, rather than to particular RAS mutants (Tanaka et al., EMBO Journal 2007), was employed for this study. The gene was synthesised by GeneArt (Life Technologies) and subsequently subcloned into the bacterial expression system pRSET to generate His-Tagged scFv. The protein was expressed in BL21 (DE3) bacterial cells and coupled to green fluorescent magnetic beads together with FITC labeled Tat penetrating peptide to facilitate the transport of the complexes into cells. A FITC labeled Gaussia luciferase export peptide was coupled, as well, to generate the MICELS complexes. The export peptide would direct the magnetic complexes to the extracellular millieu by either following the classical or an unconventional cell secretory pathway (Nickel, 2005). Any complexes that had not anchored to the activated Ras proteins, would be secreted.

An excess of 200µg scFv magnetic-protein complexes were incubated with CHO and A549 lung carcinoma cells for 1.30h. The cells were vigorously washed with PBS and trypsinised for MICELS selection (Figure 2). A large amount of background was observed during the cell selection, comprising of the cells containing the magnetic-protein complexes and an excess of free magnetic-protein complexes that had not been taken up by the cells. This excess of free magnetic-protein complexes was difficult to wash away due to their tendency of forming clumps with cells. Therefore, it was important to assess the correct concentration of magnetic-complexes that would allow for the efficient removal of the excess without compromising the cell transduction of the complexes. A serial dilution of the magnetic-protein complexes ranging from 40µg and 2.4µg were incubated with the CHO and A549 cells in a 24 well plate for 1.30h to allow them to transduce into the cells. The cells were then extensively washed with PBS to remove any complexes that had not transduced into the cells. These were further incubated for another 3h to allow for the export peptide to re-localise the complexes in the cells thereby allowing them to exit via the cell export system. This was to ensure the removal of any complexes that had not recognised the Ras antigen. The cells were then vigorously washed again before trypsinisation and selection with the magnet (Figure 3).

The cells containing the magnetic-protein complexes slowly migrated towards the magnet. These were gently washed with PBS by magnetic separation to ensure the removal of cells that did not contain the magnetic-protein complexes. The supernatant was removed and whole live cells containing activated Ras were selected using the MICELS method and placed back into culture (Figure 4). 24h later the cells had divided diluting down the cells in culture that still contained the magnetic-protein complex (Figure 4-C). The lipophilic fluorescent dye and the polysaccharid Glucuronic acid coating of the magnetic-protein complexes eventually degrade inside the cell leaving just the iron oxide core of the magnetic bead, which is non-toxic to the cells. It is thought that the iron oxide nanoparticles reside inside lysosomes (Becker et al., 2007) and are eventually degraded into iron ions via enzyme hydrolysis (Shuayev et al., 2009).

### The magnetic-protein complexes localise to subcellular compartments.

Depending on the cell signaling events undergoing in the cell, the H-Ras, N-Ras and K-Ras isoforms have all been shown to localise to the cell membrane as well as to a number of other subcellular compartments such as the ER, the Golgi, endosomes and to the perinuclear membrane (Grewal *et al.,* 2011). In this study the scFv magnetic-protein complexes localised to the cell membrane, to the perinuclear membrane and to distinct subcellular vesicles in the cytoplasm consistent with the localisation pattern of activated Ras in the A549 lung cancer cell line. Optimal results for cell selection were obtained by using 40µg of complexes. The number of cells selected decreased concomitantly with the decrease in the concentration of complexes (Figure 3-A).

A simple method is described here for selection of cells using intracellular proteins by coupling antigen-specific antibody fragments to magnetic beads and by using cell penetrating peptides to facilitate the cellular uptake of these complexes, and cell export peptides to allow the secretion of any unbound complexes via the cell secretory pathway.

The method is fast and simple, and requires minimal work. Furthermore, no cellular cytotoxicity was observed allowing the cells to be used for other downstream applications.

### References

Adjei AA (2001) Blocking oncogenic Ras signaling for cancer therapy. J Natl Cancer Inst 93: 1062-1074.
AlHajj M, Wicha MS, BenitoHernandez A, Morrison SJ, Clarke MF: Prospective identification of tumorigenic breast cancer cells. Pro Natl Acad Sci U S A 100: 39833988, 2003.
Alexiou, C., Arnold, W., Klein, R. J., Parak, F. G., Hulin, P., Bergemann, C., Erhardt, W., Wagenpfeil, S., Lubbe, A. S., Locoregional cancer treatment with magnetic drug targeting. Cancer Res. 60:6641-6648, 2000.
Avignolo C., Bagnasco L., Biasotti B., Melchiori A., Tomati V., Bauer I., Salis A., Chiossone L., Mingari M. C., Orecchia P., Carnemolla B., Neri D., Zardi L., Parodi S. Internalization via Antennapedia protein transduction domain of an scFv antibody toward c-Myc protein. The FASEB Journal 22: 1237-1245, 2008.
Becker C., Hodenius M., Blendinger G., Sechi A., Hieronymus T., Müller-Schulte D., Schmitz-Rode T., Zenke M. Uptake of magnetic nanoparticles into cells for cell tracking. J Magn Magn Mater 311:234-237, 2007.
Bigner D. D., Brown M. T., Friedman A. H., Coleman R. E., Akabani G., Friedman H. S., Thorstad W. L., McLendon R. E., Bigner S. H., Zhao X. G., Pegram C. N., Wikstrand C. J., Herndon J. N., Vick N. A., Paleologos N., Cokgor I., Provenzale J. M., Zalutsky M. R. Iodine-131-labeled antitenascin monoclonal antibody 81C6 treatment of patients with recurrent malignant gliomas: Phase I trial results. J. Clin. Oncol., 16: 2202-2212, 1998.
Bonnet D, Dick J.E. Human acute myeloid leukemia is organized as a hierarchy that originates from a primitive hematopoietic cell. Nat. Med 3: 730737, 1997.
Brinkmann U., Pai L. H., FitzGerald D. J., Willingham M., Pastan I. B3(Fv)-PE38KDEL, a single-chain immunotoxin that causes complete regression of a human carcinoma in mice. Proc. Natl. Acad. Sci. USA, 88: 8616-8620, 1991.
Brown M. T., Coleman E., Friedman A. H., Friedman H. S., McLendon R. E., Reiman R., Felsberg G. J., Tien R. D., Bigner S. H., Zalutsky M. R., Zhao X. G., Wikstrand C. J., Pegram C. N., Herndon J. N., Vick N. A., Paleologos N., Fredericks R. K., Schold S., Jr., Bigner D. D. Intrathecal 131I-labeled antitenascin monoclonal antibody 81C6 treatment of patients with leptomeningeal neoplasms or primary brain tumor resection cavities with subarachnoid communication: Phase I trial results. Clin. Cancer Res., 2: 963-972, 1996.
Chester K. A., Begent R. H., Robson L., Keep P., Pedley R. B., Boden J. A., Boxer G., Green A., Winter G., Cochet O., Hawkins R. E. Phage libraries for generation of clinically useful antibodies. Lancet, 343: 455-456, 1994.
Cinti C., Taranta M., Naldi I., Grimaldi S. Newly Engineered Magnetic Erythrocytes for Sustained and Targeted Delivery of Anti-Cancer Therapeutic Compounds. PLoS ONE 6(2): 1-9,2011.
Collins AT, Berry PA, Hyde C, Stower MJ, Maitland NJ: Prospective Identification of Tumorigenic Prostate Cancer Stem Cells. Cancer Res. 65: 1094610951, 2005.
Futaki S., Suzuki T., Ohashi W., Yagami T., Tanaka S., Ueda K., Sugiura Y. Arginine-rich peptides. An abundant source of membrane-permeable peptides having potential as carriers for intracellular protein delivery. JBiol chem. 276 (8): 5836-5840, 2001
Grewal T., Koese M., Tebar F., Enrich C. Differential Regulation of RasGAPs in Cancer. Genes & Cancer 2(3) 288-297, 2011
Hamburger AW, Salmon SE: Primary bioassay of human tumor stem cells. Science 197: 461463, 1977
Han Y., Haun Y., Deng J., Gao F., Pan B., Cui D. Expression of single-chain Fv gene specific for gamma-seminoprotein by RTS and its biological activity identification. Biotechnol Prog. 22(4):1084-9, 2006.
Houghton J, Stoicov C, Nomura S, Rogers AB, Carlson J, Li H, Cai X, Fox JG, Goldenring JR, Wang TC: Gastric cancer originating from bone marrow derived cells. Science 306: 15681571,2004.
Huston J. S., Levinson D., Mudgett-Hunter M., Tai M. S., Novotny J., Margolies M. N., Ridge R. J., Bruccoleri E. H., Crea R., Oppermann H. Protein engineering of antibody binding sites: recovery of specific activity in an anti-digoxigenin single-chain Fv analogue produced in Escherichia coli. Proc. Natl. Acad. Sci. USA, 85: 5879-5883, 1988.
Huston J. S., George A. J., Adams G. P., Stafford W. F., Jamar F., Tai M. S., McCartney J. E., Oppermann H., Heelan B. T., Peters A. M., Houston L. L., Bookman M. A., Wolf E. J., Weiner L. M. Single-chain Fv radioimmunotargeting. Q. J. Nucl. Med., 40: 320-333, 1996.
Jain R., Baxter L. Mechanisms of heterogeneous distribution of monoclonal antibodies and other macromolecules in tumors: significance of elevated interstitial pressure. Cancer Res. 48: 7022-7033, 1988.
Jeong J-G, Kim D-S, Kim Y-S, Kwon M-H. A Tat-grafted anti-nucleic acid antibody acquires nuclear-localization property and a preference for TAR RNA. Biochem. Biophys. Res. Commun. 406: 403-40, 2011.
Kaminski M. S., Zasadny K. R., Francis I. R., Milik A. W., Ross C. W., Moon S. D., Crawford S. M., Burgess J. M., Petry N. A., Butchko G. M., Glenn S. D., Wahl R. L. Radioimmunotherapy of B-cell lymphoma with [131I]anti-B1 (anti-CD20) antibody. N. Engl. J. Med., 329: 459-465, 1993.
Kaminski M. S., Zasadny K. R., Francis I. R., Fenner M. C., Ross C. W., Milik A. W., Estes J., Tuck M., Regan D., Fisher S., Glenn S. D., Wahl R. L. Iodine-131-anti-B1 radioimmunotherapy for B-cell lymphoma. J. Clin. Oncol., 14: 1974-1981,1996.
Kim CF, Jackson EL, Woolfenden AE, Lawrence S, Babar I., Vogel S, Crowley D, Bronson RT, Jacks T: Identification of bronchioalveolar stem cells in normal lung and lung cancer. Cell 121: 823-835, 2005.
Krotz, F., Wit, C., Sohn, H. Y., Zahler, S., Gloe, T., Pohl, U., Plank, C. Magnetofection-A highly efficient tool for antisense oligonucleotide delivery in vitro and in vivo. Mol. Ther. 7:700-710, 2003.
Laske D. W., Youle R. J., Oldfield E. H. Tumor regression with regional distribution of the targeted toxin TF-CRM107 in patients with malignant brain tumors. Nat. Med., 3: 1362-1368, 1997.
Lubbe, A. S., Bergemann, C., Riess, H., Schriever, F., Reichardt, P., Possinger, K., Matthias, M., Dorken, B., Herrmann, F., Gurtler, R., Hohenberger, P., Haas, N., Sohr, R., Sander, B., Lemke, A. J., Ohlendorf, D., Huhnt, W., Huhn, D. Clinical experiences with magnetic drug targeting: a phase I study with 4'-epidoxorubicin in 14 patients with advanced solid tumors. Cancer Res. 56:4686-4693, 1996.
Ma DX, Shi NQ, Qi XR. Distinct transduction modes of arginine-rich cell-penetrating peptides for cargo delivery into tumor cells. Int J Pharm. 2011.
McConnell SJ, Dinh T, Le MH, Spinella DG. Biopanning phage display libraries using magnetic beads vs. polystyrene plates. Biotechniques. 26(2):208-10, 1999.
Milenic D. E., Yokota T., Filpula D. R., Finkelman M. A. J., Dodd S. W., Wood J. F., Withlow M., Snoy P., Schlom J. Construction, binding properties, metabolism, and tumor targeting of a single chain Fv derived from the pancarcinoma monoclonal antibody CC49. Cancer Res., 51: 6363-6371, 1991.
Morrison P. F., Laske D. W., Bobo H., Oldfield E. H., Dedrick R. L. High-flow microinfusion: tissue penetration and pharmacodynamics. Am. J. Physiol., 266:R292-R305, 1994.
Nickel W. Unconventional Secretory Routes: Direct Protein Export Across the Plasma Membrane of Mammalian Cells. Traffic 6: 607-614, 2005
Niesner U., Halin C., Lozzi L., Günthert M., Neri P., Wunderli-Allenspach H., Zardi L., and Neri D. Quantitation of the Tumor-Targeting Properties of Antibody Fragments Conjugated to Cell-Permeating HIV-1 TAT Peptides. Bioconjugate Chem., 13 (4): 729-736, 2002.
Nord K, Nord O, Uhlén M, Kelley B, Ljungqvist C, Nygren PA. Recombinant human factor VIII-specific affinity ligands selected from phage-displayed combinatorial libraries of protein A. Eur. J. Biochem. 268:4269-4277, 2001
O'Brien CA, Pollett A, Gallinger S, Dick JE: A human colon cancer cell capable of initiating tumour growth in immunodeficient mice. Nature 445: 106110, 2007.
Pardal R, Clarke MF, Morrison SJ: Applying the principles of stem cell biology to cancer. Nat. Rev. Cancer 3: 895902, 2003
Pastan I. H., Archer G. E., McLendon R. E., Friedman H. S., Fuchs H. E., Wang Q-C., Pai L. H., Herndon J., Bigner D. D. Intrathecal administration of a single-chain immunotoxin, LMB-7 [B3(Fv)-PE38], produces cures of carcinomatous meningitis in a rat model. Proc. Natl. Acad. Sci. USA, 92: 2765-2769, 1995.
Press O. W., Eary J. F., Appelbaum F. R., Martin P. J., Badger C. C., Nelp W. B., Glenn S., Butchko G., Fisher D., Porter B., Matthews D. C., Fisher L. D., Bernstein I. D. Radiolabeled-antibody therapy of B-cell lymphoma with autologous bone marrow support. N. Engl. J. Med., 329: 1219-1224, 1993.
Raag R., Whitlow M. Single-chain Fvs. FASEB J., 9: 73-80, 1995.
Reisfeld R. A., Gillies S. D. Recombinant antibody fusion proteins for cancer immunotherapy. Curr. Top. Microbiol. Immunol, 213: 27-53, 1996.
Ricci Vitiani L, Lombardi DG, Pilozzi E, Biffoni M, Todaro M, Peschle C, De Maria R: Identification and expansion of human colon cancer initiating cells. Nature 445: 111115, 2007.
Saiyed ZM, Telang SD, Ramchand CN. Application of magnetic techniques in the field of drug discovery and biomedicine. Biomagn. Res. Technol. 1: 1-8, 2003.
Scherer F., Anton M., Schillinger U., Henke J., Bergemann C., Kruger A., Gansbacher B., Plank C. Magnetofection: enhancing and targeting gene delivery by magnetic force in vitro and in vivo. Gene Ther. 9:102-109, 2002.
Shuayev VI., Pisanic TR., Jun S. Magnetic nanoparticles for theragnostics. Adv Drug Deliver Rev 61:467-477, 2009.
Singh SK, Hawkins C, Clarke ID, Squire JA, Bayani J, Hide T, Henkelman RM, Cusimano MD, Dirks PB: Identification of human brain tumour initiating cells. Nature 432: 396401, 2004.
Skerra A. Bacterial expression of immunoglobulin fragments. Curr. Opin. Immunol, 5: 256-262, 1993.
Smith CA, de la Fuente J, Pelaz B, Furlani EP, Mullin M, Berry CC. The effect of static magnetic fields and tat peptides on cellular and nuclear uptake of magnetic nanoparticles. Biomaterials 31(15):4392-400, 2010.
Stark D D, Weissleder R, Elizondo G, Hahn P F, Saini S, Todd L E, Wittenberg J and Ferucci J T Superparamagnetic iron oxide: clinical application as a contrast agent for MR imaging of the liver. Radiology 168: 297-301, 1988.
Suzuki M, Honda H, Kobayashi T, Wakabayashi T, Yoshida J and Takahashi M Development of a target directed magnetic resonance contrast agent using monoclonal antibody-conjugated magnetic particles Brain Tumor Pathol. 13:127-32, 1996.
Takkinen K., Laukkanen M. L., Sizmann D., Alfthan K., Immonen T., Vanne L., Kaartinen M., Knowles J. K., Teeri T. T. An active single-chain antibody containing a cellulase linker domain is secreted by Escherichia coli. Prot. Eng., 4: 837-841, 1991.
Tanaka T, Williams RL, Rabbitts TH (2007) Tumour prevention by a single antibody domain targeting the interaction of signal transduction proteins with RAS. EMBO J. 26(13): 3250-3259
Tseng P., Carlo D., Judy J. W. Rapid and Dynamic Intracellular Patterning of Cell-Internalized Magnetic Fluorescent Nanoparticles. Nano Lett. 2009.
Vetter IR, Wittinghofer A (2001) The guanine nucleotide-binding switch in three dimensions. Science 294: 1299-1304.
Weissleder R, Cheng H C, Bogdanova A and Bogdanov A. Magnetically labeled cells can be detected by MR imaging J. Magn. Reson. Imaging 7:258-63, 1997
Winter G., Griffiths A. D., Hawkins R. E., Hoogenboom H. R. Making antibodies by phage display technology. Annu. Rev. Immunol., 12: 433-455, 1994.
Yokota T., Milenic D. E., Withlow M., Schlom J. Rapid tumor penetration of a single-chain Fv and comparison with other immunoglobulin forms. Cancer Res., 52: 3402-3408, 1992.
Yuedong Han, Yi Haun, Jinlan Deng, Feng Gao, Bifeng Pan, Daxiang Cui. Expression of Single-Chain Fv Gene Specific for γ-Seminoprotein by RTS and Its Biological Activity Identification. Biotechnology Progress 22 (4): 1084-1089, 2006

## Claims

1. A magnetic particle comprising: a ligand that specifically binds an intracellular antigen, a cell membrane penetrating cationic peptide and a cell export peptide comprising an amino acid sequence that is adapted to interact with the secretory pathway in a cell.

2. The magnetic particle according to claim 1, wherein said intracellular antigen is substantially a nuclear antigen.

3. The magnetic particle according to claim 2, wherein said nuclear antigen is selected from the group consisting of: FoxO family members, TARP, p53, Rb, E2F, hK4, BRCA1, Cdk2, SATB1, TP53INP1, Myc, Fos or Jun,

4. The magnetic particle according to claim 1, wherein said intracellular antigen is substantially a cytoplasmic antigen.

5. The magnetic particle according to claim 4, wherein said cytoplasmic antigen is selected from the group consisting of: Jak3, GATA 1-4, Stats 1-6, CBP, NFkB, IKK, PIK3CA, BRAF, PIK3CA, EML4-ALK, VEGF, EBI3, eIF2α, Akt, PI3K, IAP, Hs, Hsp, FAK, Raf, Ras, TNF, Src, Wnt or Abl.

6. The magnetic particle according to any one of claims 1-5, wherein said cationic cell membrane penetrating peptide is a natural cell penetrating peptide.

7. The magnetic particle according to any of claims 1-5, wherein said cationic cell membrane penetrating peptide is a synthetic sequence.

8. The magnetic particle according to claim 6 or 7 wherein said cationic cell membrane penetrating peptide is adapted to penetrate a mammalian cell.

9. The magnetic particle according to any one of claims 6 to 8 wherein said cationic cell membrane penetrating peptide is selected from the group consisting of: YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ or RRRRRRRRRRRR.

10. The magnetic particle according to any one of claims 1 to 13, wherein said cell export peptide is selected from the group consisting of: MCPARSLLLVATLVLLDHLSLA, MDAMKRGLCCVLLLCGAVFVSPS, MATGSRTSLLLAFGLLCLPWLQEGSA, MYRMQLLSCIALSLALVTNS or MGVKVLFALICIAVAE.

11. The magnetic particle according to any one of claims 1 to 10 wherein said ligand that binds said intracellular antigen is an antibody fragment or an aptamer.

12. The magnetic particle according to any one of claims 1-11 wherein said magnetic particle further comprises a nuclear export peptide.

13. The magnetic particle according to claim 12 wherein said nuclear export peptide comprises or consists essentially of the amino acid sequence: NELALKLAGLDINKTEGEEDAQ.

14. A method for the selection and isolation of a mammalian cell from a heterogeneous population of cells comprising:
i) forming a first preparation comprising a heterogeneous population of mammalian cells;
ii) contacting said population with a second preparation comprising a magnetic particle according to any one of claims 1-13 to provide a combined preparation and providing conditions that allow penetration and binding of said magnetic particle to an intracellular antigen;
iii) incubating said combined preparation to remove unbound magnetic particles by the secretory pathway of said mammalian cell, optionally by application of a magnetic field; and
iv) applying a magnetic field to said combined preparation to select and isolate mammalian cells comprising magnetic particles bound to an intracellular antigen.

15. A kit for customized modification of magnetic particles containing a selection of cell membrane penetrating cationic peptides selected from the group of YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ, RRRRRRRRRRRR, a selection of cell export peptides selected from the group MCPARSLLLVATLVLLDHLSLA, MDAMKRGLCCVLLLCGAVFVSPS, MATGSRTSLLLAFGLLCLPWLQEGSA, MYRMQLLSCIALSLALVTNS or MGVKVLFALICIAVAE, activation, wash and blocking/storage buffer and optionally a nuclear export peptide and a magnet.

## Patentansprüche

1. Magnetisches Teilchen, umfassend: einen spezifisch ein intrazelluläres Antigen bindenden Liganden, ein zellmembrandurchdringendes kationisches Peptid und ein Zellexportpeptid, das eine Aminosäuresequenz umfasst, die dazu angepasst ist, mit dem Sekretionsweg in einer Zelle zu interagieren.

2. Magnetisches Teilchen nach Anspruch 1, wobei das intrazelluläre Antigen im Wesentlichen ein nukleäres Antigen ist.

3. Magnetisches Teilchen nach Anspruch 2, wobei das nukleäre Antigen ausgewählt ist aus der Gruppe bestehend aus: FoxO-Familienmitgliedern, TARP, p53, Rb, E2F, hK4, BRCA1, Cdk2, SATB1, TP53INP1, Myc, Fos oder Jun.

4. Magnetisches Teilchen nach Anspruch 1, wobei das intrazelluläre Antigen im Wesentlichen ein zytoplasmatisches Antigen ist.

5. Magnetisches Teilchen nach Anspruch 4, wobei das zytoplasmatische Antigen ausgewählt ist aus der Gruppe bestehend aus: Jak3, GATA 1-4, Stats 1-6, CBP, NFkB, IKK, PIK3CA, BRAF, PIK3CA, EML4-ALK, VEGF, EBI3, elF2α, Akt, PI3K, IAP, Hs, Hsp, FAK, Raf, Ras, TNF, Src, Wnt oder Abl.

6. Magnetisches Teilchen nach einem der Ansprüche 1-5, wobei das kationische zellmembrandurchdringende Peptid ein natürliches zelldurchdringendes Peptid ist.

7. Magnetisches Teilchen nach einem der Ansprüche 1-5, wobei das kationische zellmembrandurchdringende Peptid eine synthetische Sequenz ist.

8. Magnetisches Teilchen nach Anspruch 6 oder 7, wobei das kationische zellmembrandurchdringende Peptid dazu angepasst ist, eine Säugerzelle zu durchdringen.

9. Magnetisches Teilchen nach einem der Ansprüche 6 bis 8, wobei das kationische zellmembrandurchdringende Peptid ausgewählt ist aus der Gruppe bestehend aus: YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ oder RRRRRRRRRRRR.

10. Magnetisches Teilchen nach einem der Ansprüche 1 bis 13, wobei das Zellexportpeptid ausgewählt ist aus der Gruppe bestehend aus: MCPARSLLLVATLVLLDHLSLA, MDAMKRGLCCVLLLCGAVFVSPS, MATGSRTSLLLAFGLLCLPWLQEGSA, MYRMQLLSCIALSLALSLALVTNS oder MGVKVLFALICIAVAE.

11. Magnetisches Teilchen nach einem der Ansprüche 1 bis 10, wobei der das intrazelluläre Antigen bindende Ligand ein Antikörperfragment oder ein Aptamer ist.

12. Magnetisches Teilchen nach einem der Ansprüche 1-11, wobei das magnetische Teilchen ferner ein nukleäres Exportpeptid umfasst.

13. Magnetisches Teilchen nach Anspruch 12, wobei das nukleäre Exportpeptid im Wesentlichen die Aminosäuresequenz NELALKLAGLDINKTEGEEDAQ umfasst oder im Wesentlichen aus dieser besteht.

14. Verfahren zur Auswahl und Isolierung einer Säugerzelle von einer heterogenen Zellpopulation, umfassend:
i) Bilden einer eine heterogene Säugerzellpopulation umfassenden ersten Zubereitung;
ii) Inkontaktbringen der Population mit einer ein magnetisches Teilchen nach einem der Ansprüche 1-13 umfassenden zweiten Zubereitung zum Bereitstellen einer kombinierten Zubereitung und Bereitstellen von Durchdringen und Binden des magnetischen Teilchens an ein intrazelluläres Antigen erlaubenden Bedingungen;
iii) Inkubieren der kombinierten Zubereitung zum Entfernen von ungebundenen magnetischen Teilchen durch den Sekretionsweg der Säugerzelle, wahlweise durch Anlegen eines Magnetfelds; und
iv) Anlegen eines Magnetfelds an die kombinierte Zubereitung zum Auswählen und Isolieren von Säugerzellen, die an ein intrazelluläres Antigen gebundene magnetische Teilchen umfassen.

15. Kit zur individualisierten Modifikation von magnetischen Teilchen, enthaltend eine Auswahl von zellmembrandurchdringenden kationischen Teilchen, ausgewählt aus der Gruppe YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ, RRRRRRRRRRRR, eine Auswahl an Zellexportpeptiden, ausgewählt aus der Gruppe MCPARSLLLVATLVLLDHLSLA, MDAMKRGLCCVLLLCGAVFVSPS, MATGSRTSLLLAFGLLCLPWLQEGSA, MYRMQLLSCIALSLALSLALVTNS oder MGVKVLFALICIAVAE, Aktivierungs-, Wasch- und Blockierungs-/Speicherpuffer und wahlweise ein nukleäres Peptid und einen Magneten.

## Revendications

1. Particule magnétique comprenant : un ligand qui se lie spécifiquement à un antigène intracellulaire, un peptide cationique pénétrant une membrane cellulaire et un peptide d'exportation cellulaire comprenant une séquence d'acides aminés qui est conçue pour interagir avec la voie sécrétoire dans une cellule.

2. Particule magnétique selon la revendication 1, dans laquelle ledit antigène intracellulaire étant sensiblement un antigène nucléaire.

3. Particule magnétique selon la revendication 2, dans laquelle ledit antigène nucléaire étant choisi dans le groupe constitué par : les membres de la famille FoxO, TARP, p53, Rb, E2F, hK4, BRCA1, Cdk2, SATB1, TP53INP1, Myc, Fos ou Jun.

4. Particule magnétique selon la revendication 1, dans laquelle ledit antigène intracellulaire étant sensiblement un antigène cytoplasmique.

5. Particule magnétique selon la revendication 4, dans laquelle ledit antigène cytoplasmique étant choisi dans le groupe constitué par : Jak3, GATA 1-4, Stats 1-6, CBP, NFkB, IKK, PIK3CA, BRAF, PIK3CA, EML4-ALK, VEGF, EBI3, eIF2α, Akt, PI3K, IAP, Hs, Hsp, FAK, Raf, Ras, TNF, Src, Wnt ou Abl.

6. Particule magnétique selon l'une quelconque des revendications 1 à 5, dans laquelle ledit peptide cationique pénétrant une membrane cellulaire étant un peptide naturel pénétrant une cellule.

7. Particule magnétique selon l'une quelconque des revendications 1 à 5, dans laquelle ledit peptide cationique pénétrant une membrane cellulaire étant une séquence synthétique.

8. Particule magnétique selon la revendication 6 ou 7, dans laquelle ledit peptide cationique pénétrant une membrane cellulaire étant conçu pour pénétrer une cellule de mammifère.

9. Particule magnétique selon l'une quelconque des revendications 6 à 8, dans laquelle ledit peptide cationique pénétrant une membrane cellulaire étant choisi dans le groupe constitué par : YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ ou RRRRRRRRRRRR.

10. Particule magnétique selon l'une quelconque des revendications 1 à 13, dans laquelle ledit peptide d'exportation cellulaire étant choisi dans le groupe constitué par : MCPARSLLLVATLVLLDHLSLA, MDAMKRGLCCVLLLCGAVFVSPS, MATGSRTSLLLAFGLLCLPWLQEGSA, MYRMQLLSCIALSLALVTNS ou MGVKVLFALICIAVAE.

11. Particule magnétique selon l'une quelconque des revendications 1 à 10, dans laquelle ledit ligand qui se lie audit antigène intracellulaire étant un fragment d'anticorps ou un aptamère.

12. Particule magnétique selon l'une quelconque des revendications 1 à 11, dans laquelle ladite particule magnétique comprenant également un peptide d'exportation cellulaire.

13. Particule magnétique selon la revendication 12, dans laquelle ledit peptide d'exportation cellulaire comprenant ou étant essentiellement constitué de la séquence d'acides aminés : NELALKLAGLDINKTEGEEDAQ.

14. Méthode de sélection et d'isolation d'une cellule de mammifère dans une population hétérogène de cellules consistant à :
i)former une première préparation comprenant une population hétérogène de cellules de mammifère ;
ii)mettre en contact ladite population avec une seconde préparation comprenant une particule magnétique selon l'une quelconque des revendications 1 à 13 pour obtenir une préparation mixte et réunir les conditions qui permettent la pénétration et la liaison desdites particules magnétiques à l'antigène intracellulaire ;
iii)incuber ladite préparation mixte pour éliminer toute particule magnétique non liée par la voie sécrétoire de ladite cellule de mammifère, éventuellement en appliquant un champ magnétique ; et
iv)appliquer un champ magnétique à ladite préparation mixte pour sélectionner et isoler les cellules de mammifère comprenant des particules magnétiques liées à un antigène intracellulaire.

15. Kit de modification personnalisé de particules magnétiques contenant une sélection de peptides cationiques pénétrant une membrane cellulaire choisis dans le groupe constitué par YARKKRRQRRR, YARKARRQARR, YARAAARQARA, YARAARRAARR, YARAARRAARA, YARRRRRRRRR, YAAARRRRRRR, PLSSIFSRIGDP, RQIKIWFQNRRMKWKK, WEIEDEDER, GRKKRRQRRRPQ, RRRRRRRRRRRR, une sélection de peptides d'exportation cellulaire choisis dans le groupe constitué par MCPARSLLLVATLVLLDHLSLA, MDAMKRGLCCVLLLCGAVFVSPS, MATGSRTSLLLAFGLLCLPWLQEGSA, MYRMQLLSCIALSLALVTNS ou MGVKVLFALICIAVAE, un tampon d'activation, de lavage et d'inhibition/de conservation et éventuellement un peptide d'exportation nucléaire et un aimant.


### SEQUENCE LISTING

<110> Abeterno Limited
<120> Intracellular Cell Selection
<130> 4229P/WO
<140> GB1115847.4
   <141> 2011-09-14
<160> 18
<170> PatentIn version 3.5
<210> 1
   <211> 11
   <212> PRT
   <213> Artificial Sequence
<220>
   <223> cell membrane penetrating peptide
<400> 1
   Tyr Ala Arg Lys Lys Arg Arg Gln Arg Arg Arg
   1               5                   10
<210> 2
   <211> 11
   <212> PRT
   <213> Artificial Sequence
<220>
   <223> cell membrane penetrating peptide
<400> 2
   Tyr Ala Arg Lys Ala Arg Arg Gln Ala Arg Arg
   1               5                   10
<210> 3
   <211> 11
   <212> PRT
   <213> Artificial Sequence
<220>
   <223> cell membrane penetrating peptide
<400> 3
   Tyr Ala Arg Ala Ala Ala Arg Gln Ala Arg Ala
   1               5                   10
<210> 4
   <211> 11
   <212> PRT
   <213> Artificial Sequence
<220>
   <223> cell membrane penetrating peptide
<400> 4
   Tyr Ala Arg Ala Ala Arg Arg Ala Ala Arg Arg
   1               5                   10
<210> 5
   <211> 11
   <212> PRT
   <213> Artificial Sequence
<220>
   <223> cell membrane penetrating peptide
<400> 5
   Tyr Ala Arg Ala Ala Arg Arg Ala Ala Arg Ala
   1               5                   10
<210> 6
   <211> 11
   <212> PRT
   <213> Artificial Sequence
<220>
   <223> cell membrane penetrating peptide
<400> 6
   Tyr Ala Arg Arg Arg Arg Arg Arg Arg Arg Arg
   1               5                   10
<210> 7
   <211> 11
   <212> PRT
   <213> Artificial Sequence
<220>
   <223> cell membrane penetrating peptide
<400> 7
   Tyr Ala Ala Ala Arg Arg Arg Arg Arg Arg Arg
   1               5                   10
<210> 8
   <211> 12
   <212> PRT
   <213> Artificial Sequence
<220>
   <223> cell membrane penetrating peptide
<400> 8
   Pro Leu Ser Ser Ile Phe Ser Arg Ile Gly Asp Pro
   1 5 10
<210> 9
   <211> 16
   <212> PRT
   <213> Artificial Sequence
<220>
   <223> cell membrane penetrating peptide
<400> 9
   Arg Gln Ile Lys Ile Trp Phe Gln Asn Arg Arg Met Lys Trp Lys Lys
   1 5 10 15
<210> 10
   <211> 9
   <212> PRT
   <213> Artificial Sequence
<220>
   <223> Cell membrane penetrating peptide
<400> 10
   Trp Glu Ile Glu Asp Glu Asp Glu Arg
   1 5
<210> 11
   <211> 12
   <212> PRT
   <213> Artificial Sequence
<220>
   <223> cell membrane penetrating peptide
<400> 11
   Gly Arg Lys Lys Arg Arg Gln Arg Arg Arg Pro Gln
   1 5 10
<210> 12
   <211> 12
   <212> PRT
   <213> Artificial Sequence
<220>
   <223> cell membrane penetrating peptide
<400> 12
   Arg Arg Arg Arg Arg Arg Arg Arg Arg Arg Arg Arg
   1 5 10
<210> 13
   <211> 22
   <212> PRT
   <213> Artificial Sequence
<220>
   <223> secretory peptide
<400> 13
   Met Cys Pro Ala Arg Ser Leu Leu Leu Val Ala Thr Leu Val Leu Leu
   1               5                   10                  15
   Asp His Leu Ser Leu Ala
               20
<210> 14
   <211> 23
   <212> PRT
   <213> Artificial Sequence
<220>
   <223> secretory peptide
<400> 14
   Met Asp Ala Met Lys Arg Gly Leu Cys Cys Val Leu Leu Leu Cys Gly
   1               5                   10                  15
   Ala Val Phe Val Ser Pro Ser
               20
<210> 15
   <211> 26
   <212> PRT
   <213> Artificial Sequence
<220>
   <223> secretory peptide
<400> 15
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala
               20                  25
<210> 16
   <211> 20
   <212> PRT
   <213> Artificial Sequence
<220>
   <223> secretory peptide
<400> 16
   Met Tyr Arg Met Gln Leu Leu Ser Cys Ile Ala Leu Ser Leu Ala Leu
   1               5                   10                  15
   Val Thr Asn Ser
               20
<210> 17
   <211> 16
   <212> PRT
   <213> Artificial Sequence
<220>
   <223> secretory peptide
<400> 17
   Met Gly Val Lys Val Leu Phe Ala Leu Ile Cys Ile Ala Val Ala Glu
   1               5                   10                  15
<210> 18
   <211> 22
   <212> PRT
   <213> Artificial Sequence
<220>
   <223> Nuclear export peptide
<400> 18
   Asn Glu Leu Ala Leu Lys Leu Ala Gly Leu Asp Ile Asn Lys Thr Glu
   1               5                   10                  15
   Gly Glu Glu Asp Ala Gln
               20